# EUROPEAN PATENT APPLICATION

(11) **EP 2 019 134 A1**
(43) Date of publication of application: **28.01.2009**
(21) Application number: 07014711.1
(22) Date of filing: 26.07.2007
(51) Int. Cl.: C12N 9/02, A61K 48/00

(54) **Gene therapy of chronic granulomatous disease**

(71) Applicant: Vision 7 GmbH, 22335 Hamburg (DE); Chemotherapeutisches Forschungsinstitut Georg-Speyer-Haus, 60596 Frankfurt (DE)
(72) Inventor: Stein, Stefan, Dr., 63808 Haibach (DE); Grez, Manuel, Dr., 69121 Heidelberg (DE); Schambach, Axel, 21220 Seevetal (DE)
(74) Representative: UEXKÜLL & STOLBERG

(57) **Abstract**

The present invention relates to the field of gene therapy. In particular, it provides a nucleic acid encoding gp91phox, wherein codon-usage of said gp91phox nucleic acid is optimized for expression in human cells, and an expression construct, in particular a retroviral vector suitable for gene therapy of chronic granulomatous disease, comprising said nucleic acid. Additionally, methods of preparing cells comprising the construct, said cells and pharmaceutical compositions comprising the construct or cells of the invention are provided.

## Description

The present invention relates to the field of gene therapy. In particular, it provides a nucleic acid encoding gp91phox, wherein codon-usage of said gp91phox nucleic acid is optimized for expression in human cells, and an expression construct, in particular a retroviral vector suitable for gene therapy of chronic granulomatous disease, comprising said nucleic acid. Additionally, methods of preparing cells comprising the construct, said cells and pharmaceutical compositions comprising the construct or cells of the invention are provided.

Chronic granulomatous disease (CGD) is a severe inherited imnunodeficiency caused by a defect in the oxidative antimicrobial activity of phagocytes resulting from absence or malfunction of the respiratory burst oxidase normally expressed in neutrophils and other phagocytic leukocytes. Two-thirds of the patients are males hemizygous for mutations in the X-linked gene encoding gp91phox. The disease is characterized by recurrent, often life threatening bacterial and fungal infections.

Gp91phox is a highly glycosylated transmembrane protein with 570 amino acids that forms a heterodimer with p22 phox and interacts with other oxidase subunits for functional activity in the generation of superoxide (Roos et al., Chronic granulomatous disease. In Ochs et al (eds.) Primary immunodeficiency diseases. New York, NY, Oxford University Press, 1999).

If regular care and conventional therapy fail, the disease can be cured by bone marrow transplantation. This treatment is, however, only available to patients with human leukocyte antigen-identical sibling or matched unrelated donors. One therapeutic option for patients lacking suitable donors is the genetic modification of autologous hematopoietic stem cells.

The disease can be treated by gene therapy. A vector carrying the gene gp91phox is used in a clinical study and leads to significant clinical results (Ott et al., Nat Med. 2006, 12:386-8). However, concerns about possible side effects, e.g. regarding cancerogenesis by insertion mutagenesis, are an issue. Thus, at the moment, treatment is reserved for fatally ill patients without any other therapeutic options.

Thus, there is a need in the art to provide a novel vector with comparable activity and a lower risk of insertion mutagenesis.

Self inactivating vectors (SIN vectors) are known to minimize the risks of insertion mutagenesis. The safety features of current SIN vectors can be summarized as follows:
- The extensive U3 (LTR, long terminal repeat) deletion abolishes viral enhancer/promoter activity, preventing the synthesis of full-length vector RNA in target cells and minimizing the risk for the generation of replication-competent retroviruses.
- The elimination of the LTR enhancer sequences precludes the activation of a promoter located adjacent from the vector integration site.
- The SIN design prevents potential interference between the viral LTRs and the internal promoter.
- Virus production is achieved by co-transfecting separate plasmids expressing the viral proteins and the RNA that is to be packaged, which greatly reduces the possibility of recombination events to assemble replication competent viruses.
- Alternatively, virus production is achieved from stable packaging cell lines.

SIN vectors are described in Kraunus J et al. (Self-inactivating retroviral vectors with improved RNA processing. Gene Ther. 2004;11(21):1568-78).

However, expression of gp91phox in SIN vectors failed, as no adequate titers for infection of cells in the context of gene therapy of humans were achieved. Minimum titers are considered to be 5x10e5 to 5x10e6.

The skilled person was thus faced with the problem of developing a nucleic acid for expression of gp91phox in viral and non-viral expression systems for therapy of chronic granulomatosis, which minimizes the risks associated with gene therapy, in particular the risk of insertion mutagenesis, and which is produced in adequate titers for gene therapy.

This problem is solved by provision of a nucleic acid encoding gp91phox, wherein codon-usage of said gp91phox nucleic acid is optimized. In particular, if gene therapy of humans is of interest, gp91phox is human gp91phox and the codon usage is optimized for expression in human cells.

In essence, in the optimized sequence, rarely used codons of the wild type gp91phox sequence (SEQ ID NO: 2) are exchanged for codons more frequently used in the cell of interest. Optimization can be measured according to the codon-adaptation index (CAI), which can be calculated according to a method published by Fuglsang, A., Codon optimizer: a freeware tool for codon optimization. Protein Expr Purif. 2003;31(2):247-9.

In human wildtype gp91phox, the CAI is 0.77. In the nucleic acid of the invention, the codon-adaptation index is at least 0.85, preferably at least 0. 90, at least 0.95, more preferably at least 0.98 or at least 0.99.

In a preferred embodiment of the invention, the nucleic acid encoding gp91phox has been further optimized for expression in human cells, by incorporating one, several or all of the following changes.

The GC content of the wildtype nucleic acid encoding gp91phox is 47%. While the GC content does not have to be changed, it is preferred that it is 47-75%, preferably 50-70%, more preferably 55-65% in the optimized construct. In a particularly preferred embodiment, the GC content of the nucleic acid encoding gp91phox is 61%.

Furthermore, the invention provides nucleic acids encoding gp91phox wherein, in comparison to wildtype gp91phox, less cryptic poly-A sites are present in the nucleic acid encoding gp91phox. Cryptic poly-A sites are sequences that under certain circumstances can be recognized as poly-A addition sites. Cryptic poly A sites in wildtype human gp91phox are located at positions 477-482 and 759-764. Preferably, the nucleic acid encoding gp91phox does not contain any cryptic poly-A sites.

Additionally or alternatively, in comparison to wildtype gp91phox, less consensus or cryptic splice donor/acceptor sites are present in the nucleic acid encoding gp91phox. Cryptic splice donor sites are sequences that under certain circumstances can be recognized as splice donor sites. Consensus (cryptic), splice donor/acceptor sites in wildtype human gp91phox are located between positions 850-857 and between positions 92-99. Preferably, the nucleic acid encoding gp91phox does not contain any cryptic splice donor sites.

In a preferred embodiment of the invention, less RNA instability motifs are present in the nucleic acid encoding gp91phox in comparison to wildtype gp91phox, or no RNA instability motifs are present in the nucleic acid. RNA instability motifs are RNA sequences which reduces the half life span of mRNAs. RNA instability motifs in wildtype human gp91phox are located at positions 360-364 and 303-308.

Preferably, the nucleic acid encoding gp91phox has a CAI of at least 0.90, in particular of 0.99, a GC content of 55-65%, in particular of 61%, and some or all cryptic poly-A sites, cryptic splice donor sites and RNA instability motifs are deleted. In a particularly preferred embodiment, the nucleic acid encoding gp91phox has the sequence according to SEQ ID NO: 1 (gp91phox opt).

The present invention also provides an expression construct comprising the nucleic acid of the invention encoding gp91phox. The expression construct can be viral or non-viral.

In the expression construct, the nucleic acid of the invention encoding gp91phox is operably linked to a promoter functional in human cells, e.g., expression of gp91phox can be effected from this promoter in human cells. In a preferred embodiment, the promoter is a tissue-specific promoter, in particular, a myelospecific promoter. Thus, expression in cells not playing a role in chronic granulomatous disease is much reduced, leading to a higher specificity of treatment. In the context of gene therapy, it is not preferred to use strong, non-tissue specific promoters for expression of gp91phox, as a significantly higher clonal transformation has been found in cell systems.

In a preferred embodiment, the invention provides a vector comprising the nucleic acid of the invention encoding gp91phox, e.g., a lentivirus or gammaretrovirus vector. Preferentially the vector can be based on HIV, SIV or SIN gamma retroviral vectors. In a particularly preferred embodiment, the vector is derived from a murine retrovirus. It is preferred that the vector is a self-inactivating (SIN) vector, as described above. For example, the vector can be a monocistronic or bicistronic SIN retroviral vector. In all of these vectors, use of the optimized gp91phox sequence significantly enhances titers and expression of the transgene. Especially in the context of vectors with weak, tissue-specific promoters, it has surprisingly been found that use of an optimized gp91phox sequence of the invention is essential for an expression of the transgene that allows treatment of chronic granulomatous disease.

In a preferred embodiment, the vector of the invention has the structure
Δ -R-U5-SD-P-gp91phox-WPRE-Δ-R-U5. Δ represents deletion of the enhancer region within the viral LTRs,
R represents repetitive sequences at the 5' and 3'LTRs,
U5 represents unique 5' region,
SD represents splice donor,
P represents any promoter, preferably, a myelospecific promoter like c-fes or MRP8,
WPRE represents woodchuck hepatitis post-transcriptional regulatory element.

One vector providing a backbone suitable for the invention is SERS11.SF.eGFP.pre.

Gp91phox is any of the synthetic nucleotides encoding gp91phox described above, preferably, the gp91phox sequence according to SEQ ID NO: 1 (gp91phox opt). Thus, the vector can be SERS11.SF.gp91s.W or SERS11.fes.gp91s.W, which are described in detail below. Advantageously, this vector can be stably produced by a cell clone. The vector of the invention is suitable for gene therapy, leading to expression of gp91phox in a human subject.

In one aspect, the invention thus provides a method for the preparation of a cell expressing gp91phox, wherein the cell is transfected with an expression construct, in particular, a retroviral vector of the invention.

The cell preferably is of human origin. Preferably, the cell is a hematopoietic cell, in particular a primary cell isolated from a subject. In the context of gene therapy, autologous cells are preferred, so the subject preferably has chronic granulomatous disease. For example, peripheral blood CD34+ progenitor cells (CD34+PBPC, CD34+ stem cell) can be isolated from peripheral blood, e.g. by apharesis of G-CSF-mobilized peripheral blood. CD34+ PBPC can be pre-stimulated for 36 hours in X-VIVO 10 medium + 2 mM L-glutamine, supplemented with IL-3 (60 ng/ml), SCF (300 ng/ml), Flt3-L (300 ng/ml), and TPO (100 ng/ml). Transduction can be done on days 2 and 3. On day 4 cells can be treated with hG-CSF (10 ng/µl) to induce granulocytic differentiation.

However, transfected cell lines, e.g. K562 or HEK 293T cells, can also be useful in the context of the invention.

Cells comprising the expression construct of the invention or transfected with the vector of the invention can be employed for the preparation of a pharmaceutical composition for the therapy of chronic granulomatous disease. The invention also relates to a method of treating chronic granolamatous disease by gene therapy, wherein a subject with chronic granolamatous disease is contacted with the expression construct/vector of the invention or cells comprising the same.

Pharmaceutical compositions comprising the construct, in particular the retroviral vector, or the cells according to the invention are also provided. Such pharmaceutical compositions may comprise suitable carrier and excipients, which are well known in the state of the art.

The invention for the first time allows gene therapy of patients with chronic granulomatous disease with a construct that minimizes side effects, e.g., the risk of insertional mutagenesis, and as a consequence, of carcinogenesis induced by gene therapy. Gene therapy thus becomes an option for other groups of patients in addition to fatally ill patients, who are already successfully treated with vectors for expression of gp91phox. The high titers and good expression rates achieved with the constructs of the invention will also be of great benefit to patients.

The invention is described below with reference to specific examples to better illustrate the invention. However, these examples are not to be construed to limit the scope of the invention. All references cited above are herewith fully incorporated in the specification.

### Figures

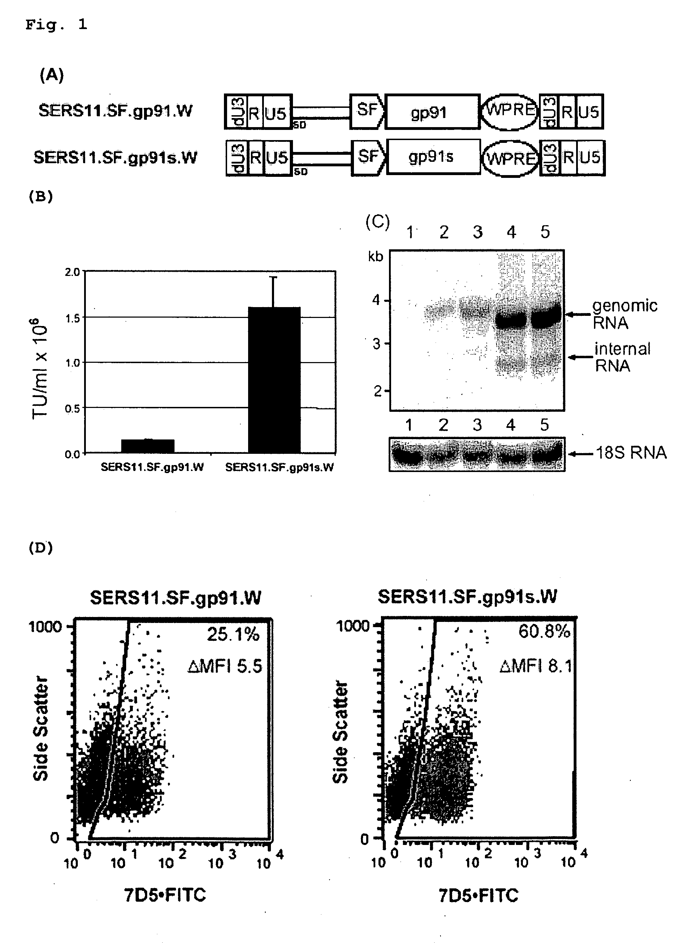

**Figure 1****.** Codon optimization of gp91phox leads to increase viral titers and gp91phox expression. (A). Structure of the monocistronic gamma SIN retroviral vectors used. (B). Titer of the vectors shown in A. Titers were determined on X-CGD PLB985 cells with serial dilution of viral supernatants by FACS. The mean ± standard deviation of at least three independent experiments is shown. (C). 293T cells were transfected in duplicate with SERS11.SF.gp91.W (lanes 2 and 3) or SERS11.SF.gp91s.W (lanes 4 and 5). 24 hours later total RNA was extracted from transfected cells run on a formaldehyde gel, blotted and hybridized against a WPRE probe. (D). Expression of gp91phox in X-CGD PLB985 cells transduced with SERS11.SF.gp91.W or SERS11.SF.gp91s.W.

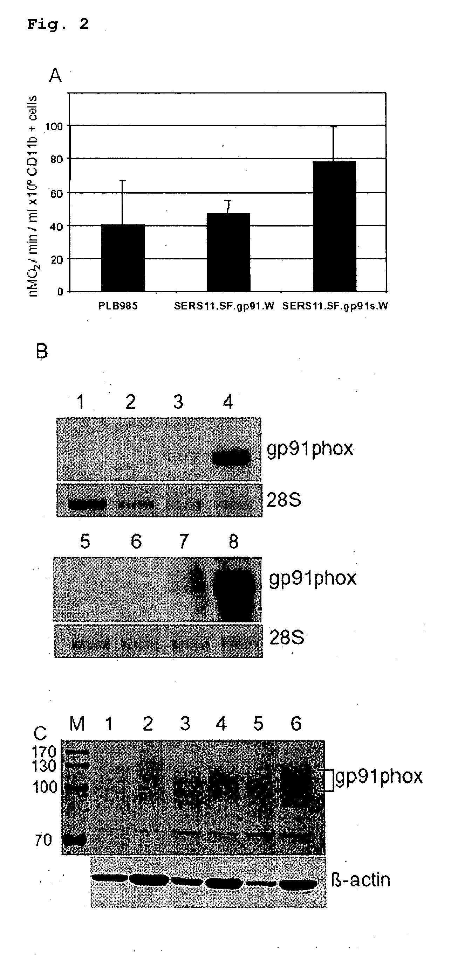

**Figure 2****.** Reconstitution of superoxide production is improved in X-CGD cells expressing the codon-optimized gp91phox cDNA. (A). X-CGD PLB985 cells transduced with SERS11.SF.gp91.W or SERS11.SF.gp91s.W were sorted for gp91phox expressing cells. Granulocytic differentiation was induced with DMSO for 6 days and superoxide activity was measured by the continuous cytochrome C reduction assay. (B). Total protein extracts were obtained from untransduced wild type X-CGD PLB985 cells (1 and 2), or cells transduced with SERS11.SF.gp91.W (3 and 4) or SERS11.SF.gp91s.W (5 and 6) before (1, 3 and 5) or after DMSO differentiation (2, 4, and 6). 20 µg of each extract were separated on an SDS-polyacrylamide gel and gp91phox proteins were visualized after hybridization with the gp91phox specific monoclonal antibody m48.

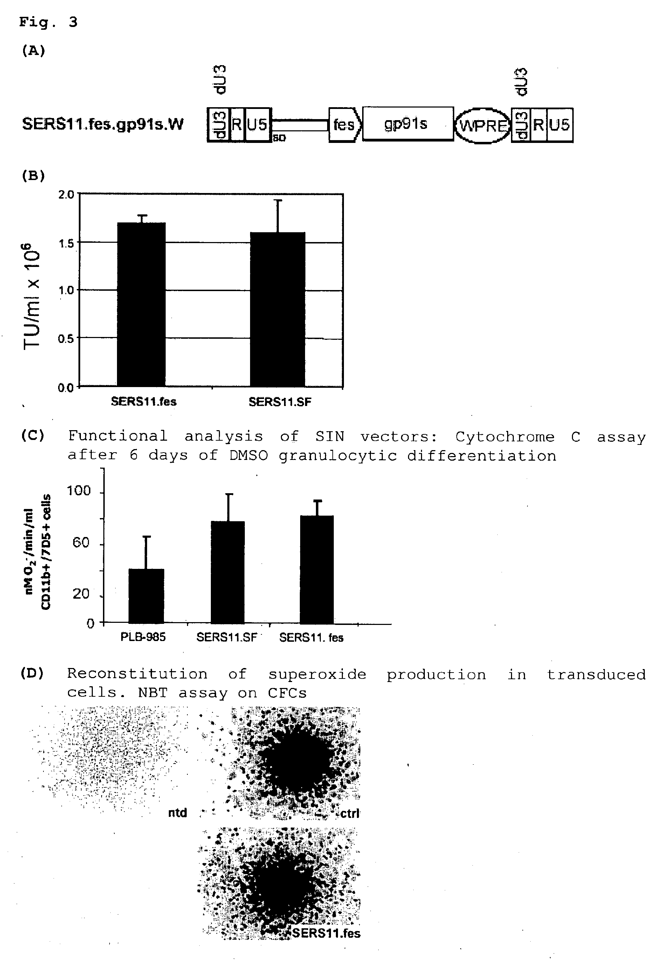

Figure 3. Efficacy of the SERS11.fes.gp91s.W SIN retroviral vector in human hematopoietic cells.
   A. Structure of the SERS11.fes.gp91s.W retroviral vector. This vector is similar to SERS11.SF.gp91s.W but contains the huam nc-fes promoter instead of the SF promoter. B. Titer of the SERS11.fes.gp91s.W (SERS11.fes) retroviral vector on X-CGD PLB985 cells in comparison to the titer of the SERS11.SF.gp91s.W (SERS11.S.F) vector. C. Reconstitution of superoxide activity in granulocytes derived from transduced X-CGD PLB-985 cells after granulocytic differentiation. Superoxide activity was assessed by the cytochrome C assay. PLB-985 wild type cells; SERS11.SF, X-CGD PLB985 cells transduced with SERS11.SF.gp91s.W; SERS11.fes, X-CGD PLB985 cells transduced with SERS11.fes.gp91s.W. D. Reconstitution of superoxide activity in human X-CGD bone marrow cells after transduction with SERS11.fes.gp91s.W. Reconstitution of superoxide activity was assessed by the NBT assay.

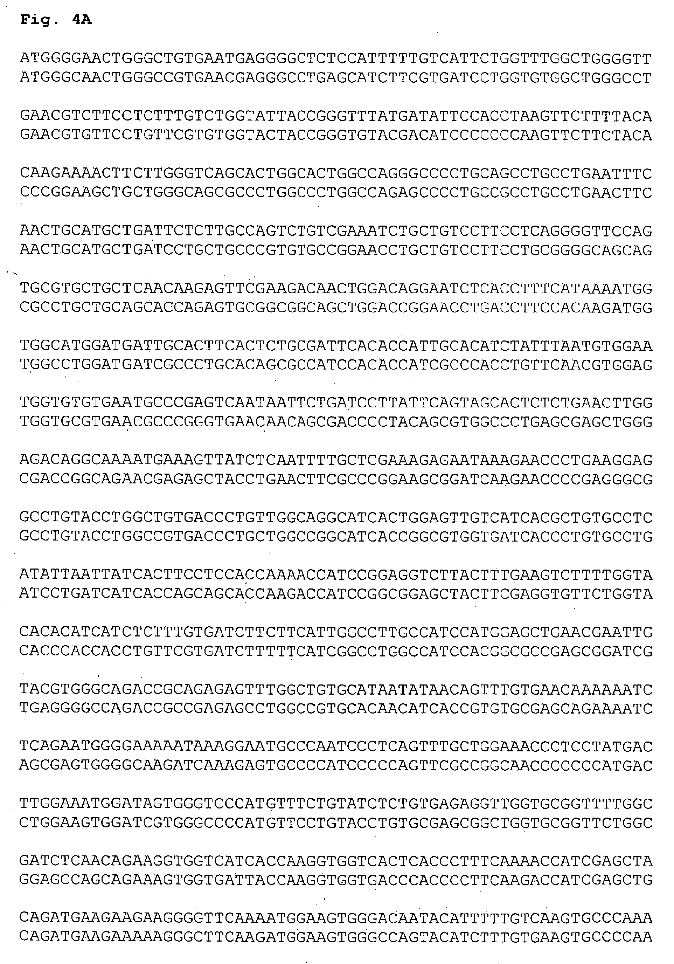

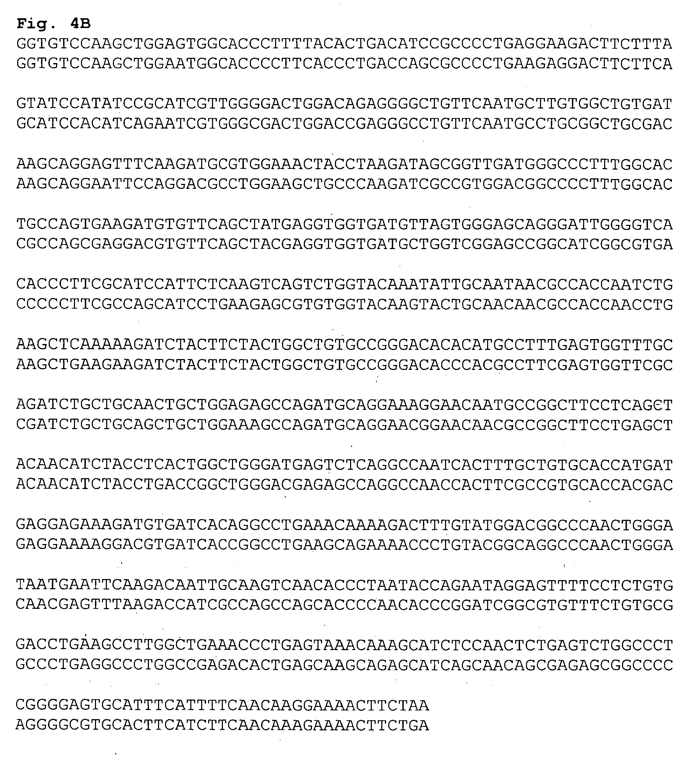

**Figure 4****.** A comparison of the sequence of wildtype gp91phox (top line) and synthetic, optimized gp91phox (gp91s, bottom line) is shown.

### EXAMPLES

### Materials and methods

### Constructs

SERS11.SF.gp91.W was constructed by replacing the GFP gene in SERS11.SF.eGFP.pre (Schambach et al., [1]) with the wild type gp91phox cDNA. Thereafter the pre element in SERS11.SF.gp91.pre was replaced with the WPRE sequences described in (Schambach et al., [2]) to generate SERS11.SF.gp91.W. Lastly, the wild type gp91phox sequence in SERS11.SF.gp91.W was replaced with the synthetic gp91phox cDNA to generate SERS1.SF.gp91s.W. SERS11.fes.gp91s.W was generated by exchanging the SF promoter with the human fes promoter in SERS11.SF.gp91s.W. All cloning steps were done by conventional molecular biology methodology.

### Virus production and titer

Virus production was done as described in Schambach et al. [1]. The titer of the viral supernatants was determined on X-CGD PLB985 cells by transducing 3x10e5 cells with increasing dilutions of viral supernatant (0.5 ml). The amount of transduced cells was determined 3 days later by FACS using the gp91phox monoclonal antibody 7D5. Titer (TU/ml) calculations were done according to the formula (transduced cells [%] /100) x seeded cells x dilution factor x 2.

### NBT and Cytochrome C assays, Western Blot

NBT and Cytochrome C assays and Western Blot were carried out following methods known in the state of the art.

### Cells and culture conditions

Autologous peripheral blood stem cells (PBSC) can be harvested after subcutaneous G-CSF stimulation. CD34⁺ progenitor cells can be immunomagnetically selected on CliniMacs columns (Miltenyi Biotec, Bergisch Gladbach, Germany) according to the manufacturer instructions. CD34+ cells can be cultured in X-VIVO 10 medium + 2 mM L-glutamine, supplemented with IL-3 (60 ng/ml), SCF (300 ng/ml), Flt3-L (300 ng/ml), and TPO (100 ng/ml). HEK 293T and X-CGD PLB985 cells are cultured in DMEM supplemented with 10 % fetal bovine serum (FBS) and 100 U/ml penicillin/streptomycin, and 2 mM glutamine.

### Transduction and flow cytometric analysis

CD34⁺ cells can be prestimulated for 36 hours in X-VIVO 10 medium + 2 mM L-glutamine, supplemented with IL-3 (60 ng/ml), SCF (300 ng/ml), Flt3-L (300 ng/ml), and TPO (100 ng/ml). Transduction can be performed in tissue culture flasks coated with Retronectin (Takara, Otsu, Japan) and preloaded with retroviral vector containing supernatant as described in [3]. This procedure can be repeated several times. 24 hours after the final transduction, the cells are harvested and analyzed for phenotype and gene transfer efficiency.

Human gp91^{phox} expression was determined by direct staining with the murine anti-human monoclonal antibody 7D5. For comparable analyses of expression levels and transgene function equally low MOI were used for all virus vectors.

### Functional analyses of gp91^{phox} transgenes

Reconstitution of NADPH oxidase activity in neutrophils after gene transfer can be assessed by oxidation of dihydrorhodamine 123 [4], reduction of nitrobluetetrazolium [5], or reduction of cytochrome C [6] according to standard protocols.

### References.

1. Schambach A, Mueller D, Galla M, Verstegen MM, Wagemaker G, Loew R, Baum C, Bohne J. Overcoming promoter competition in packaging cells improves production of self-inactivating retroviral vectors. Gene Ther. 2006, 13:1524-1533.
2. Schambach A, Bohne J, Baum C, Hermann FG, Egerer L, von Laer D, Giroglou T. Woodchuck hepatitis virus post-transcriptional regulatory element deleted from X protein and promoter sequences enhances retroviral vector titer and expression. Gene Ther. 2006, 13:641-645.
3. Kuhlcke, K. et al. Highly efficient retroviral gene transfer based on centrifugation-mediated vector preloading of tissue culture vessels. Mol Ther 5, 473-478 (2002).
4. Vowells, S.J., Sekhsaria, S., Malech, H.L., Shalit, M. & Fleisher, T.A. Flow cytometric analysis of the granulocyte respiratory burst: a comparison study of fluorescent probes. J Immunol Methods 178, 89-97 (1995).
5. Bohler, M.C. et al. A study of 25 patients with chronic granulomatous disease: a new classification by correlating respiratory burst, cytochrome b, and flavoprotein. J Clin Immunol 6, 136-145 (1986) .
6. Mayo, L.A. & Curnutte, J.T. Kinetic microplate assay for superoxide production by neutrophils and other phagocytic cells. Methods Enzymol 186, 567-575 (1990).

### Results

### Gamma SIN monocistronic gp91phox vectors.

Retroviral vectors containing an optimized backbone architecture including deletion of enhancer and TATA box (SIN configuration) were cloned for the expression of gp91phox in hematopoietic cells (Figure 1A). However initial attempts to generate a gamma SIN retroviral vectors expressing the wild type version of gp91phox failed because the titer of the virus produced by transient transfection protocols were very low (< 5x10⁵ TU/ml, Figure 1B). The inclusion of elements known to enhance titer like WPRE or splicing signals did not increase titer (data not shown).

Therefore we considered to perform a codon optimization of the gp91phox cDNA. An analysis of the gp91phox wild type mRNA sequence indicated several motifs which could interfere with expression of gp91phox in human cells. Based on this analysis, a complete new gp91phox cDNA was synthesized. In essence, rare used codons were exchanged to increase the "codon adaptation index (CAI)", a parameter that describes how well the codons match the codon usage preference of the target organism. The CAI was optimized from originally 0.77 (native gp91phox) to 0.99 (codon-optimized gp91phox). Furthermore the GC content was increased from originally 47% to 61%. In addition putative polyA sites, cryptic splice donor sites and RNA instability motifs were removed. The synthetic, codon-optimized gp91phox (gp91s) was used to replace the native gp91phox cDNA in the gamma SIN retroviral vector SERS11.SF.gp91.W to generate SERS11.SF.gp91s.W (Figure 1A). Viral supernatants were produced from both constructs by transient transfection on 293T cells and virus titers were determined on the X-CGD PLB985 cell line. Inclusion of the codon-optimized gp91phox into the gamma SIN backbone surprisingly led to a 10fold increase in titer (Figure 1B). This effect was reproducible in a series of others constructs.

Indeed 293T cells transfected with SERS11.SF.gp91s.W contained 4-6 times more genomic viral RNA than cells transfected with SERS11.SF.gp91.W (Figure 1C). Finally, X-CGD PLB985 cells transduced with the vector expressing the codon-optimized gp91phox cDNA showed increase surface expression of the glycoprotein as determined by FACS and increased mean fluorescence intensity (Figure 1D).

### Higher reconstitution of superoxide production in cells expressing the optimized gp91phox.

X-CGD PLB985 cells were transduced at low MOI (< 3) to achieve a mean of 1-2 proviral copies per cell. Transduced cells (usually less than 15%) were enriched by immunomagnetic selection to >95% gp91phox positive cells. Transgene copy was estimated by semi-quantitative PCR for both population and found to contain equal mean copy numbers per cell (1-1.2 copies per cell each). Subsequently granulocyte differentiation was induced with DMSO for 6 days and subsequently NADPH oxidase activity was assessed by reduction of ferri cytochrome C to its ferrous form. Superoxide production in cells transduced with SERS11.SF.gp91s.W was two fold higher than that observed in cells transduced with vectors expressing native gp91phox (Figure 2A). This increase in superoxide production was the result of increase gp91phox RNA and protein levels in SERS11.SF.gp91s.W transduced and differentiated cells (Figures 2B and 2C). A quantitative assessment of the amount of gp91phox protein in transduced and differentiated cells showed a 2fold increase in protein levels in cells transduced with the codon-optimized version of gp91phox.

A SIN gamma retroviral vector containing a myeloid specific promoter and the synthetic gp91phox sequences for the gene therapy of chronic granulomatous disease.

Ideally, internal cellular promoters rather than viral promoters should be preferred for therapeutic purposes. We use a 507 bp sequence derived from the human c-fes promoter to direct the expression of gp91phox to myeloid cells. This vector SERS11.fes.gp91s.W (Fig. 3A) showed similar titers to SERS11.SF.gp91s.W on X-CGD PLB985 cells (Fig. 3B) and was able to reconstitute superoxide activity in X-CGD PLB985 to high levels (Fig. 3C). Moreover, transduction of human bone marrow cells derived from an X-CGD patient with SERS11.fes.gp91s.W resulted in high levels of superoxide activity in granulocytes derived from the transduced bone marrow cells. Thus the SERS11.fes.gp91s.W vector combines the safety aspects of the SIN architecture with a high expression of the synthetic gp91phox cDNA and thus is the vector of choice for a clinical application.

SEQUENCE LISTING

<110>  Vision-7 GmbH et al.
<120>  Gene therapy of chronic granulomatous disease
<130>  P 73831
<160>  2
<170>  PatentIn version 3.3

<210>  1
<211>  1713
<212>  DNA
<213>  Homo sapiens

<400>  1
   atgggcaact gggccgtgaa cgagggcctg agcatcttcg tgatcctggt gtggctgggc      60
   ctgaacgtgt tcctgttcgt gtggtactac cgggtgtacg acatcccccc caagttcttc     120
   tacacccgga agctgctggg cagcgccctg gccctggcca gagcccctgc cgcctgcctg     180
   aacttcaact gcatgctgat cctgctgccc gtgtgccgga acctgctgtc cttcctgcgg     240
   ggcagcagcg cctgctgcag caccagagtg cggcggcagc tggaccggaa cctgaccttc     300
   cacaagatgg tggcctggat gatcgccctg cacagcgcca tccacaccat cgcccacctg     360
   ttcaacgtgg agtggtgcgt gaacgcccgg gtgaacaaca gcgaccccta cagcgtggcc     420
   ctgagcgagc tgggcgaccg gcagaacgag agctacctga acttcgcccg gaagcggatc     480
   aagaaccccg agggcggcct gtacctggcc gtgaccctgc tggccggcat caccggcgtg     540
   gtgatcaccc tgtgcctgat cctgatcatc accagcagca ccaagaccat ccggcggagc     600
   tacttcgagg tgttctggta cacccaccac ctgttcgtga tctttttcat cggcctggcc      660
   atccacggcg ccgagcggat cgtgaggggc cagaccgccg agagcctggc cgtgcacaac     720
   atcaccgtgt gcgagcagaa aatcagcgag tggggcaaga tcaaagagtg ccccatcccc     780
   cagttcgccg gcaacccccc catgacctgg aagtggatcg tgggccccat gttcctgtac     840
   ctgtgcgagc ggctggtgcg gttctggcgg agccagcaga aagtggtgat taccaaggtg     900
   gtgacccacc ccttcaagac catcgagctg cagatgaaga aaaagggctt caagatggaa     960
   gtgggccagt acatctttgt gaagtgcccc aaggtgtcca agctggaatg gcaccccttc    1020
   accctgacca gcgcccctga agaggacttc ttcagcatcc acatcagaat cgtgggcgac    1080
   tggaccgagg gcctgttcaa tgcctgcggc tgcgacaagc aggaattcca ggacgcctgg    1140
   aagctgccca agatcgccgt ggacggcccc tttggcaccg ccagcgagga cgtgttcagc    1200
   tacgaggtgg tgatgctggt cggagccggc atcggcgtga ccccctttgc cagcatcctg     1260
   aagagcgtgt ggtacaagta ctgcaacaac gccaccaacc tgaagctgaa gaagatctac    1320
   ttctactggc tgtgccggga cacccacgcc ttcgagtggt tcgccgatct gctgcagctg    1380
   ctggaaagcc agatgcagga acggaacaac gccggcttcc tgagctacaa catctacctg    1440
   accggctggg acgagagcca ggccaaccac ttcgccgtgc accacgacga ggaaaaggac    1500
   gtgatcaccg gcctgaagca gaaaaccctg tacggcaggc ccaactggga caacgagttt    1560
   aagaccatcg ccagccagca ccccaacacc cggatcggcg tgtttctgtg cggccctgag    1620
   gccctggccg agacactgag caagcagagc atcagcaaca gcgagagcgg ccccaggggc    1680
   gtgcacttca tcttcaacaa agaaaacttc tga                                 1713

<210>  2
<211>  1713
<212>  DNA
<213>  Homo sapiens

<400>  2
atggggaact gggctgtgaa tgaggggctc tccatttttg tcattctggt ttggctgggg      60
ttgaacgtct tcctctttgt ctggtattac cgggtttatg atattccacc taagttcttt     120
tacacaagaa aacttcttgg gtcagcactg gcactggcca gggcccctgc agcctgcctg     180
aatttcaact gcatgctgat tctcttgcca gtctgtcgaa atctgctgtc cttcctcagg     240
ggttccagtg cgtgctgctc aacaagagtt cgaagacaac tggacaggaa tctcaccttt     300
cataaaatgg tggcatggat gattgcactt cactctgcga ttcacaccat tgcacatcta     360
tttaatgtgg aatggtgtgt gaatgcccga gtcaataatt ctgatcctta ttcagtagca     420
ctctctgaac ttggagacag gcaaaatgaa agttatctca attttgctcg aaagagaata     480
aagaaccctg aaggaggcct gtacctggct gtgaccctgt tggcaggcat cactggagtt     540

gtcatcacgc tgtgcctcat attaattatc acttcctcca ccaaaaccat ccggaggtct 600
tactttgaag tcttttggta cacacatcat ctctttgtga tcttcttcat tggccttgcc 660
atccatggag ctgaacgaat tgtacgtggg cagaccgcag agagtttggc tgtgcataat 720
ataacagttt gtgaacaaaa aatctcagaa tggggaaaaa taaaggaatg cccaatccct 780
cagtttgctg gaaaccctcc tatgacttgg aaatggatag tgggtcccat gtttctgtat 840
ctctgtgaga ggttggtgcg gttttggcga tctcaacaga aggtggtcat caccaaggtg 900
gtcactcacc ctttcaaaac catcgagcta cagatgaaga agaaggggtt caaaatggaa 960
gtgggacaat acatttttgt caagtgccca aaggtgtcca agctggagtg gcaccctttt 1020
acactgacat ccgcccctga ggaagacttc tttagtatcc atatccgcat cgttggggac 1080
tggacagagg ggctgttcaa tgcttgtggc tgtgataagc aggagtttca agatgcgtgg 1140
aaactaccta agatagcggt tgatgggccc tttggcactg ccagtgaaga tgtgttcagc 1200
tatgaggtgg tgatgttagt gggagcaggg attggggtca caccettcgc atccattctc 1260
aagtcagtct ggtacaaata ttgcaataac gccaccaatc tgaagctcaa aaagatctac 1320
ttctactggc tgtgccggga cacacatgcc tttgagtggt ttgcagatct gctgcaactg 1380
ctggagagcc agatgcagga aaggaacaat gccggcttcc tcagctacaa catctacctc 1440
actggctggg atgagtctca ggccaatcac tttgctgtgc accatgatga ggagaaagat 1500
gtgatcacag gcctgaaaca aaagactttg tatggacggc ccaactggga taatgaattc 1560
aagacaattg caagtcaaca ccctaatacc agaataggag ttttcctctg tggacctgaa 1620
gccttggctg aaaccctgag taaacaaagc atctccaact ctgagtctgg ccctcgggga 1680
gtgcatttca ttttcaacaa ggaaaacttc taa 1713

## Claims

1. Nucleic acid encoding gp91phox, wherein codon-usage of said gp91phox nucleic acid is optimized for expression in human cells.

2. Nucleic acid according to claim 1, wherein the codon-adaptation index (CAI) is at least 0.85, preferably at least 0.90, more preferably at least 0.99.

3. Nucleic acid according to claims 1 or 2, wherein the GC content of said nucleic acid encoding gp91phox is 47-75%, preferably 50-70%, more preferably 55-65%.

4. Nucleic acid according to claim 3, wherein the GC content is 61%.

5. Nucleic acid according to claims 1 to 4, wherein in comparison to wildtype gp91phox less cryptic poly-A sites are present in the nucleic acid encoding gp91phox.

6. Nucleic acid according to claim 5, wherein the nucleic acid encoding gp91phox does not contain cryptic poly-A sites.

7. Nucleic acid according to claims 1 to 6, wherein in comparison to wildtype gp91phox less cryptic splice donor sites are present in the nucleic acid encoding gp91phox.

8. Nucleic acid according to claim 7, wherein the nucleic acid encoding gp91phox does not contain cryptic splice donor sites.

9. Nucleic acid according to claims 1 bis 8, wherein in comparison to wildtype gp91phox less RNA instability motifs are present in the nucleic acid encoding gp91phox.

10. Nucleic acid according to claim 9, wherein the nucleic acid encoding gp91phox does not contain RNA instability motifs.

11. Expression construct, wherein the nucleic acid according to claims 1 to 10 is operably linked to a promoter functional in human cells.

12. Construct according to claim 11, wherein the promoter is a tissue-specific promoter.

13. Construct according to claim 12, wherein the promoter is a myelospecific promoter.

14. Construct according to claims 11 to 13, wherein the construct is a viral vector.

15. Construct according to claim 14, wherein the vector is a gammaretroviral vector.

16. Construct according to claims 14 or 15, wherein the vector is a self-inactivating (SIN) vector.

17. Construct according to claim 16, wherein the vector is a monocistronic or bicistronic SIN retroviral vector.

18. Construct according to claim 16, wherein the vector has the structure
Δ -R-U5-SD-P-gp91phox-WPRE-Δ-R-U5.

19. Construct according to claims 11 to 18, wherein the construct comprises the sequence according to SEQ ID NO: 1.

20. Construct according to claims 18 or 19, wherein the vector is SERS11.SF.gp91s.W or SERS11.fes.gp91s.W.

21. Method for the preparation of a cell expressing gp91phox, wherein the cell is transfected with a construct according to claims 11 to 20.

22. Cell comprising the construct according to claims 11 to 20.

23. Cell according to claim 22, wherein the cell is a hematopoietic cell.

24. Use of the construct according to claims 11 to 20 or the cell according to claims 22 or 23 for the preparation of a pharmaceutical composition for the therapy of chronic granulomatosis.

25. Pharmaceutical composition comprising the construct according to claims 11 to 20 or the cells according to claims 22 or 23.
